# EUROPEAN PATENT SPECIFICATION

(11) **EP 1 904 773 B1**
(45) Date of publication and mention of the grant of the patent: **14.11.2012**
(21) Application number: 06772174.6
(22) Date of filing: 06.06.2006
(51) Int. Cl.: A61F 2/80, F16K 15/06, F16K 15/20, F16K 27/02

(54) **EXPULSION VALVE ASSEMBLY**
AUSTRITTSVENTILBAUGRUPPE
ENSEMBLE DE VALVE D EXPULSION

(30) Priority: 28.06.2005 US 168956; 28.11.2005 US 287647
(43) Date of publication of application: 02.04.2008
(73) Proprietor: Otto Bock HealthCare GmbH, 37115 Duderstadt (DE)
(72) Inventor: ALLEY, Randall, Thousand Oaks, CA 91360 (US); SOSS, Adam, Winetka, CA 91306 (US); BOUCH, Dustin, Petaluma, CA 94952 (US)
(74) Representative: Lins, Edgar
(86) International application number: PCT/US2006/021766
(87) International publication number: WO 2007/001745

(56) References cited:
- US-A- 3 913 615
- US-A- 5 653 256
- US-A- 6 063 125
- US-A- 6 079 445
- US-B1- 6 287 345

## Description

### FIELD OF THE INVENTION

The present invention relates to check valves and in particular to latched and magnetic valve assemblies used to maintain a preferred suspension pressure at a prosthetic socket or interface between a limb and a prosthesis, among other applications.

### BACKGROUND OF THE INVENTION

The maintenance of a negative pressure or suction in a prosthetic socket or at a related interface to thereby facilitate a desired limb suspension is typically achieved through the use of an expulsion-type check valve (e.g. auto, manual or both). Such valves are typically configured to provide a threaded base or seat. The base, in turn, normally supports a threaded valve insert that is configured to ensure a proper airtight seal.

Proper placement of the valve insert relative to the base requires a high degree of dexterity and hand-eye coordination, especially when used in a prosthetic limb. That is, most typically the valve base is located at the most distal aspect of a limb socket. The base is typically thermoformed or laminated into the socket. Such a location can be difficult to see, but can also present an awkward relative alignment angle to mount or detach the valve insert.

Because the valve threads, which exhibit a relatively fine pitch, have to line up perfectly in order for the valve insert to be set properly, the combination of poor visibility and high demands for physical dexterity typically result in difficult donning situations for individuals with sound hands and fingers. Removal of the valve can present similar challenges, as the quality of the seal is often related to how tight the insert has been screwed into the valve base. A significant amount of friction can result from overzealous tightening of the valve insert, making it extremely difficult to remove.

For individuals with an involved upper extremity or extremities (e.g. injured, deformed, diseased or the result of insufficient congenital development), the insertion and/or removal of the valve insert from the valve seat can prove impossible. This circumstance is particularly onerous for the debilitated user who has to periodically self-adjust the limb.

US 5,653,256 discloses a charge valve assembly consisting of a valve stem that can be machined from a cylindrical metal stock. The valve stem is provided with a large diameter central bore and a base portion where the bore communicates with a pressurized fluid delivery passage for transferring pressurized fluid to the interior of a housing for an automotive air conditioning component or other pressurized applications. The bore defines a valve cavity adapted to receive a cartridge valve assembly provided at its upper end with a valve seat which is engaged by a popper valve. Said valve establishes one-way flow between the central bore and an intake port for the charge fluid. The upper end of the bore is provided with a C-clip retainer groove which is adapted to receive a C-clip retainer by which the cartridge valve assembly, after assembling in place in the bore, is retained within the valve stem. The C-clip retainer is inserted by using a conventional tool. An easy withdrawing of the cartridge container from the valve stem is not intended and not possible without a tool by which the C-clip retainer may be compressed and removed from the C-clip retainer groove.

The foregoing difficulties have been overcome with the present valve assembly, which in one construction provides a threadless base that is adapted to receive a mating, latched insert. The valve assembly at the coupling junction between the base and insert is particularly constructed to provide for a latched connection. The interconnection provides a sliding, sealed valve insert piece that is respectively pushed or pulled from the seat or base during mounting and removal. The connection is maintained or broken with cooperating latch arms that assure proper insert retention.

In another construction, a threadless base supports an insert via O'rings and a magnetic coupling. A spring biased valve piece or stem piece having adjustable air pressure release capabilities is concentrically supported in the insert.

### SUMMARY OF THE INVENTION

It is a primary object of the present invention to provide a threadless valve assembly wherein a valve base or seat supports a mating insert that contains a valve or stem piece.

It is a further object of the invention to provide a valve assembly having a threadless base.

It is a further object of the invention to provide a valve assembly having a base containing a bore that accepts a slide mounted insert.

It is a further object of the invention to provide a latched interconnection between the valve seat and the insert.

It is a further object of the invention to provide a valve assembly that accepts an insert having pivoting latch arms.

It is a further object of the invention to provide a magnetic coupling between the valve seat and the insert.

It is a further object of the invention to provide a valve assembly wherein O'ring(s) seal a valve insert within a seat bore.

It is a further object of the invention to provide a resiliently biased check valve piece within the valve insert.

It is a further object of the invention to provide a pressure adjustable check valve piece within the valve insert.

The foregoing objects are achieved in a presently preferred valve assembly, having the features of claim 1 or claim 13. The threadless insert is pushed into the base by hand or by using a tool or other appliance that imparts sufficient pressure on the valve insert to overcome an internal resilient tension and seal friction.

The valve seat and valve insert include overlapping, annular flange surfaces. A seal (e.g. an O'ring) seals the insert within the seat. A concentric, resiliently biased stem or check valve piece is retained in the insert with a second seal. Resiliently biased retainers or latches (e.g. latch arms) are mounted to the insert and located to pivot during insert insertion and extraction. Latch surfaces at provided arms flex outward during insertion and spring back to a concentric, restraining alignment with the base upon traversing an interconnecting annular flange at the base.

During insert removal, portions of the latch arms are depressed and pivoted to disconnect the arms from the base. The insert can then be removed, which action is facilitated if the insert is independently biased with other resilient devices (e.g. springs) fitted between the insert and base. Although two latch arms are symmetrically arranged to the present base, a single latch arm or other arrangements of pivot mechanisms can be used.

A plunger-type stem or check valve piece is used to allow auto or manual expulsion of air or other gas or liquid from the limb socket, although other types and mountings of the stem piece can also be adapted to the insert. Similarly, the stem piece might be interconnected to the insert in a related manner, such as with latch arms or other threadless restraints. Although the present valve assembly finds particular application with prosthetic limbs, it is to be appreciated the valve might be used in other applications.

In another construction, the valve insert is magnetically coupled to the base. A pair of seals (e.g. O'rings) facilitate retention and provide a seal action. An adjustable, resiliently biased stem or check valve piece is supported to the insert with another seal (e.g. O'ring). A set screw cooperates with the insert and a spring to provide a pressure adjustable one-way check valve action.

Still other objects, advantages and constructions of the present invention, among various considered improvements and modifications, will become apparent from the detailed description provided hereinafter. It should be understood that the detailed description and specific examples, while indicating a presently preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein similar reference callouts are used at the various figures, and wherein:
FIG. 1 shows a perspective view to a prosthetic limb that has been adapted to include one construction of a push valve assembly of the invention.
FIG. 2 shows an exploded drawing to the foregoing push valve assembly.
FIG. 3 shows a cross section drawing to the valve assembly at an initial insertion position or just prior to complete removal of the insert from the valve base.
FIG. 4 shows a cross section drawing to the valve assembly with the latch arms squeezed toward one another in preparation for full insertion or removal.
FIG. 5 shows a cross section drawing to the valve assembly with the latch arms latched closed to contain a concentric expulsion valve piece fitted within the valve base.
FIG. 6 shows an exploded assembly drawing in perspective view to a magnetic valve assembly containing a concentric expulsion valve fitted within a threadless valve base.

### DETAILED DESCRIPTION OF THE PREFERRED EMBODIMENTS

The following description of the preferred embodiment is merely exemplary in nature and is in no way intended to limit the invention, its application, or uses. For purposes of clarity, the same reference numbers are used throughout the drawings to identify similar components.

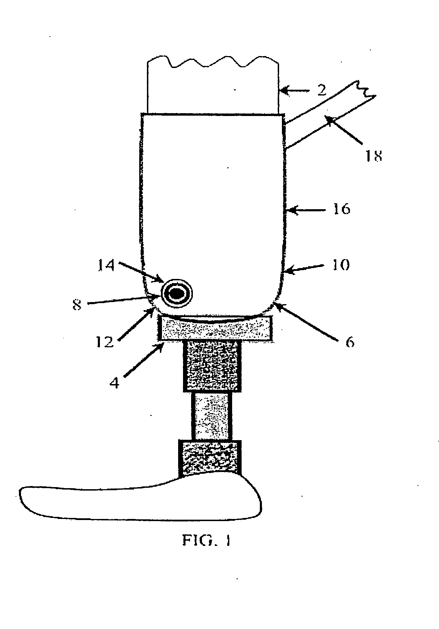

Referring to Figure 1 a view is shown to a coupling between a limb 2 (e.g. a leg or arm) and a suitable prosthetic appliance 4. A cutaway view is shown to a limb socket 6 at the appliance 4 and where a valve 8 constructed in accord with the subject invention is shown. The valve 8 is fitted to a socket assembly 10 that is used at the interface between the appliance 4 and an extremity of the user's limb 2. The valve 8 is mounted to a socket piece 12 that is shaped to mate with a surgically prepared distal end of the limb extremity. A valve base 14 is typically thermoformed or laminated into the socket piece 12.

A collar or harness 16 is formed to contain the socket piece 12 and shelter the distal end of the extremity. Suitable padding 16 and/or straps 18 are provided at the collar 14 to enhance the durability of the attachment.

Depending upon the limb and application, a principal concern is to maintain a limb to prosthesis connection that provides maximum flexibility without causing limb ulceration. Various interfaces have been developed that use padding materials such as foams, plastics, polyester and acrylic resins etc. Interfaces that incorporate air, gas or liquid buffers or cushions or combinations thereof are also frequently used. It is with respect to prosthetic devices that use gas and/or liquid buffer materials that the valve 8 of the invention finds particular application.

The valve 8 is particularly used at included ports to allow the filling of the socket piece 12 with a suitable gas or liquid interface material between the socket piece 12 and limb extremity. The valve 8 also serves as a check valve to prevent the uncontrolled expulsion of the interface material.

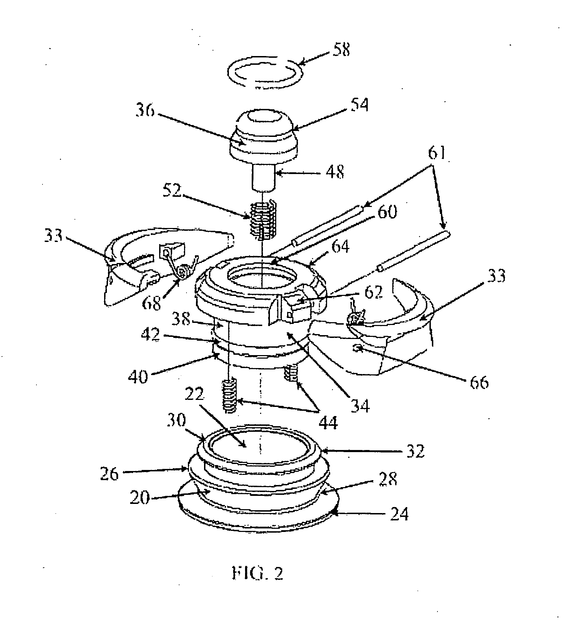

With attention to Figure 2, the valve 8 is shown in exploded assembly. The valve 8 includes a base 20 that is designed to exhibit a suitable geometric configuration adapted to a preferred mounting of the valve 8. Presently, the base 20 is generally cylindrical in shape, although could exhibit a hexagonal, octagonal or other shape.

A bore 22 longitudinally extends through the base 20. A ringed flange 24 projects from the base 8 and can be laminated to or thermoformed in a socket piece 12 with a variety of suitable adhesives and tapes. The diameter of the flange 24 can be varied as desired.

An adjoining tapered flange surface 26 is also provided that forms a "V", "U" or other suitably shaped groove 28 relative to the flange 24. The groove 28 accommodates valve retention within a thermoformed mounting. Suitable grooves, ridges, knurling or other types of projections and the like can be provided at the surface 26 and groove 28 to facilitate retention to the appliance 4.

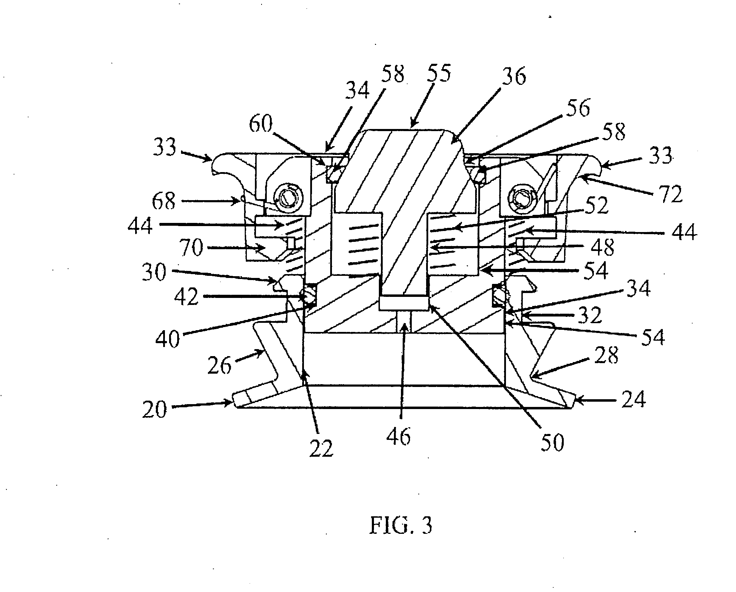

A third flanged surface 30 is provided that defmes a groove 32 relative to the outermost portion of the flange 26. The groove 32 mates with and contains a pair of latch arms or members 33 that pivot from an associated valve insert 34 to secure the insert 34 and a valve piece 36 at the insert to the bore 22. The details of the mounting of the insert 34 and valve piece 36 are more apparent from the following description with respect to the section views at Figures 3, 4 and 5. The flange surfaces 26 and 30 are preferably circularly continuous although can be segmented into a number of discontinuous sections. The construction of the pivoting members 33 (e.g. latch arms) can also be varied

The insert 34 provides a generally cylindrical body 38 that is sized to closely mate with the diameter of the bore 22. An annular groove 40 contains an O'ring seal 42. The seal 42 prevents the migration of a gas or liquid contained within the socket piece 12 through the bore 22 and past the insert 34. A pair of resilient members 44 (e.g. springs) depend from the body 38 and normally bias the insert 34 to an elevated condition. Although the base 20 and insert 34 are shown in cylindrical shapes, other shapes or combinations of shapes can be adapted as desired. The insert 34 might also be constructed to index to a preferred alignment with the base 20. The springs 44 may comprise other resilient devices, elastomers or other compressible/expansible members. The springs 44 are supported at their top by recessed holes that receive and maintain the alignment of each spring 44 and are supported at their base by resting on the top edge of the valve base 14.

A port 46 is aligned to the longitudinal center of the body 38, reference Figure 3. A stem 48 of the valve piece 36 is separately restrained to the insert 34 at a bore 50 to control liquid or gas flow through the port 46 and bore 50. A spring 52 is supported about the stem 48 in a bore 54 and biases a circular, tapered surface 56 at a head 55 of the valve piece 36 to seal against an O'ring 58 contained at a groove 60 within the bore 54.

The valve piece 36 thus acts as a check valve and prevents flow through the bores 54, 50 and port 46, unless the valve piece 36 is depressed from contact with the O'ring seal 58. It is to be appreciated a variety of other resilient devices and materials can be used to bias the valve piece 36. Similarly, different seat/seal arrangements can be configured between the valve piece 36 and bore 54 to control flow through the bores 46, 50 and 54. The valve piece 36 may also be mounted such that it is normally depressed against the port 46 versus being raised above the port 46, such as with the aid of a snap ring (not shown) and spring 52 mounted to bias the stem 48 to engage the port 46.

The insert 34 is restrained to the base 20 with the latch arms 33 which are mounted to pivot about pivot pins 61. The pins 61 mount through segmented portions 62 of a flange 64 that radiates from the sidewalls of the insert 34 and nest within aligned bores 66 in the arms 33. A torsion spring 68 is fitted around each pin 60 and between the flange 64 and arms 33. The ends of the springs 68 are retained to induce flanges 70 that project from the ends of the arms 33 to pivot inward. Thus, the flanges 70 are restrained beneath the flange 30 in the base 20 and within the groove 32, once the insert 34 is fully seated within the base 20. Although two latch arms 33 are presently used, a single arm or additional arms or other pivoting restraints might alternatively be incorporated into the assembly 8. The latch arm(s) 33 can exhibit any variety of shapes and may also be constructed of flexible materials such that pivot pins may not be required. The flanges 70 might also be restrained to depressions or projections at the base 20.

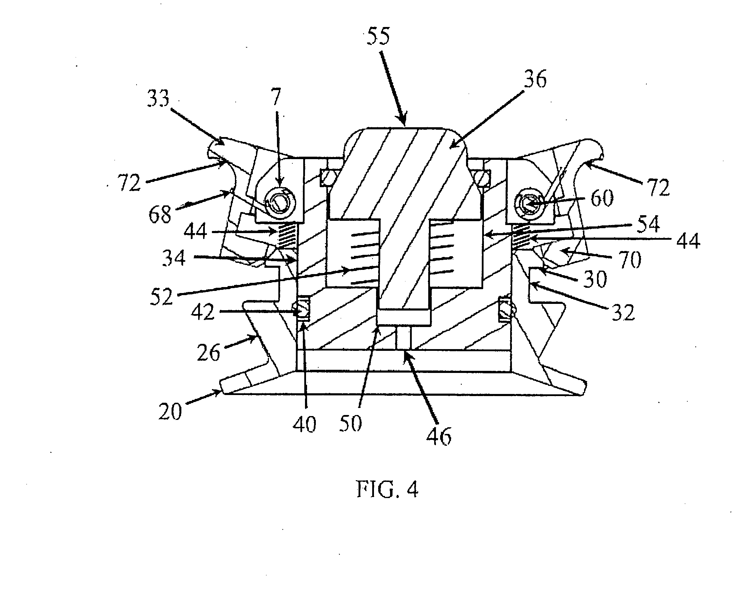

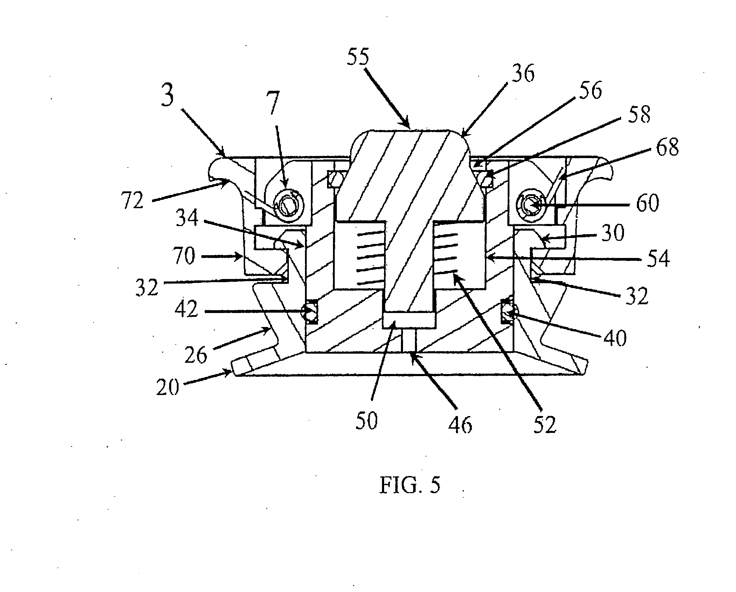

In the latter regard, attention is drawn to Figures 3, 4 and 5 and wherein the flanges 70 at the arms 33 are respectively shown in a depressed condition at Figure 3, a partially flexed condition at Figure 4 and a seated condition at Figure 5. During normal mounting, the insert 34 is merely aligned to the base 20 and the tapers at the flanges 70 and 30 induce the flange arms 33 to pivot outward, reference Figures 3 and 4. Once the insert 34 is pushed past the flange 30, the flanges 70 are biased inward and are captured in the groove 32, reference Figure 5.

The valve piece 36 mounts by suitably lubricating the piece 36 and depressing it and the spring 52 into the bore 54. Removal of the insert 34 is effected by manually lifting up on the finger depressions 72. A typical user of a prosthesis 4 having an air or liquid interface material is thereby now able to readily fit and make suitable adjustments to enhance the fit and comfort of the prosthesis without the inconveniences previously experienced.

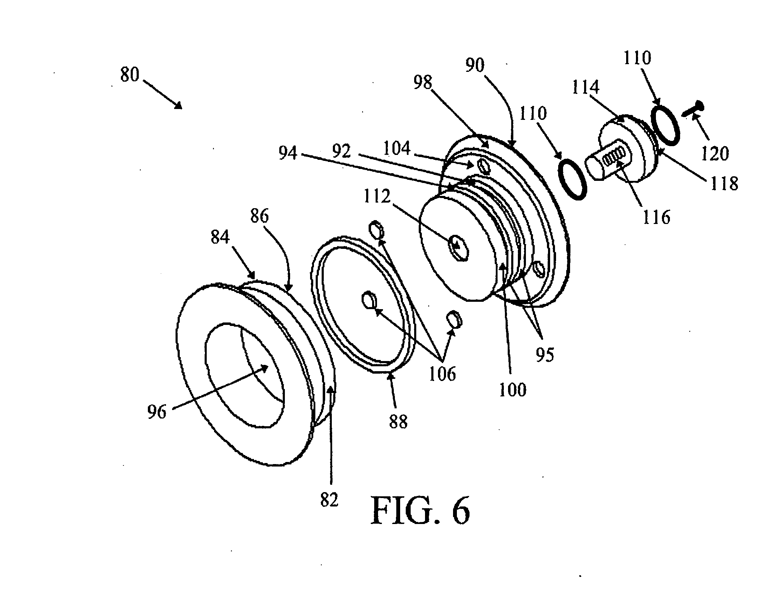

Appreciating the efficacy of the improved valve assembly 8 and with attention to FIG. 6, another threadless valve assembly 80 is shown in exploded assembly. The assembly 80 includes a seat or base 82 constructed of a suitable thermoplastic, resin or other biocompatible material that can be molded or laminated into place in a suitable socket assembly 10. The seat 82 is similar to the seat 20 except lacks a flange 30 and groove 32. Instead, a raised surface 84 includes a groove 86 that supports a ferromagnetic ring 88 or other suitable magnetically compatible structure that couples to a valve insert 90.

The ring 88 can be constructed of a variety of magnetically compatible materials. Alternatively and among some of other variations, the seat 82 and/or insert 90 can be constructed of a magnetically compatible material, the surface 84 or insert 90 can include segmented, ferromagnetic sections or can be impregnated with iron or similar particles.

The valve insert 90 mounts within the seat 82 and supports upper and lower seals 92 and 94 (e.g. O'rings) that cooperate with a bore 96. A flange 98 radiates outward from a stem 100 that contains the O'rings 92 and 94 in grooves 95. The O-rings 92 and 94 prevent the passage of air or other buffer material between the insert 90 and seat 82.

Displaced around a surface 104 of the flange 98 are a number of magnets 106 that are mounted to optimally interact with the ring 88 or other magnetic structure at the seat 82. Alternatively and as with the ring 88 and in lieu of magnets 106, the flange 98 can be constructed of a ferromagnetic material, can be impregnated with iron or similar particles or can include segmented ferromagnetic sections that are mounted in registry with the ring 88 or other magnetically compatible material.

A check valve assembly 108 mounts to the insert 90 and cooperates with an O'ring seal 110 mounted in a bore 112 of the insert 90 to provide for a one-way flow of the buffer material. The assembly 108 includes a plunger/stem or valve piece 114 and a spring 116 that is supported to resiliently interact with the insert 90 and maintain a flanged cap/valve surface 118 in contact with the O-ring 110.

A setscrew 120 extends through the stem 114 and is threaded to the insert 90. The screw 120 can be adjusted to control the tension at the spring 116 and contact tension between the cap 118 and seal 110. The pressure necessary to overcome the seal can thereby be regulated to automatically provide for a preferred one-way flow (e.g. inside to outside) of the buffer material (e.g. air, other gas or liquid).

From the foregoing, it is to be appreciated the described construction of the complete valve assemblies 8 and 80 are merely exemplary of a class of presently preferred valves. From the suggested modifications and others that may be apparent to those skilled in the art, it also is to be appreciated the invention can be implemented in still other configurations. Selected portions of the valve assemblies may also be adapted into other valve assemblies. The invention is defined by the appended claims.

## Claims

1. A valve comprising
(a) a base (20) having i) a cylindrical body and through which a bore (22) extends, ii) a first sealing surface located within said bore (22), and iii) a threadless latch surface;
(b) an insert (34) having a body adapted to mount in the bore (22) of said base (20) and to engage said first sealing surface, wherein said insert body includes a flow bore (50) having a second sealing surface within the flow bore (50), wherein fastener means (33) fitted to insert body mates with the threadless latch surface which includes a flange (30) radiating from the base body and a depression (32) displaced from said flange (30), and wherein the fastener means includes an arm (33) having a flanged surface (70) and being mounted to move relative to the insert body such that the flanged surface (70) mates with the depression (32) at said body whereby said insert (34) can be inserted, restrained and withdrawn from said base (20) upon appropriately manipulating said arm (33);
(c) a valve piece (36) having a stem (48) to mount in the bore (50) of said insert (34) and wherein a surface of said valve piece (36) mates in sealing engagement with the second sealing surface and said valve piece (36) may be depressed from contact with said second sealing surface so as to open a passage through the flow bore (50).

2. A valve as set forth in claim 2 **characterized by** a plurality of resilient members (44) mounted to said insert (34) to a retention condition in said body and bias said valve piece (36) into sealed engagement with said second sealing surface.

3. A valve as set forth in one of the claims 1 or 2, **characterized by** a plurality of springs (44) mounted bias said insert (34) in the bore (22) of the base body and said valve piece (36) in the bore (50) of the insert body.

4. A valve as set forth in one of the claims 1 or 3 **characterized by** a spring (68) mounted to resiliently bias said arm (33) to pivot as said insert (34) is mounted in the bore (22) of said base (20) and return said arm (33) to a retention condition once said flanged surface (70) is aligned to said depression (32).

5. A valve as set forth in one of the claims 1 to 4, **characterized in that** said first and second arms (33) are resiliently biased to flex and return to a retention condition when the flanged surfaces (70) of said first and second arms (33) engage said depression (32).

6. A valve as set forth in one of the claims 1 to 5, **characterized in that** said first and second seals (42, 58) comprise O'rings.

7. A valve as set forth in one of the claims 1 to 6, **characterized in that** a mounting flange (24) is annular and exhibits a diameter greater than a diameter of the base body.

8. A valve as set forth in claim 7, **characterized in that** the base body includes an annular groove (32) and wherein said insert (34) includes first and second resiliently biased arms (33) which are biased to flexibly pivot and return to a retention condition when flanged surfaces (70) of said first and second arms (33) engage said groove (32) and thereby restrain said insert (34) to said body.

9. A valve as set forth in one of the claims 1 to 8, **characterized in that** the first sealing surface is a first O'ring seal (42) mounted within the bore (22) of the body of base (20), and **in that** the second sealing surface includes a second O'ring seal (58) mounted within the flow bore (50).

10. A valve as set forth in claim 9 **characterized by** a spring (44) for biasing said valve piece (36) and to abutment with said second O'ring (58).

11. A valve as set forth in one of the claims 1 to 10, **characterized in that** the depression includes a groove (32).

12. A valve as set forth in claim 10 or 11, **characterized by** a plurality of resilient members (44) biasing said insert (34) to retention condition in said body and biasing said valve piece (36) into sealed engagement with said second O'ring seal (58).

13. A valve comprising
(a) a base (82) having i) a body and through which a bore (96) extends, and ii) a first seal (92, 94) mounted within said bore (96);
(b) an insert (90) having a body adapted to mount in the bore (96) of said (82) and engage said first seal (92, 94), wherein said insert body includes the flow bore (112) having a second seal (110) mounted within in the flow bore (112); and
(c) a valve piece (114) having a stem adapted to mount in the bore (112) of said insert (90) and wherein a surface of said valve piece (114) mates and sealing engagement with the second seal (110), wherein the base has a magnetic portion (88),
the insert has a magnetic portion (106), wherein the magnetic portion (106) of the insert (90) is mounted to interact with the base magnetic portion (88) and couple said insert (90) to said base (82) whereby said insert (90) can be inserted, restrained and withdrawn from said base (82) upon appropriately overcoming the magnetic attraction between the coupled base (82) and insert (90).

14. A valve as set forth in claim 13, **characterized in that** said insert (90) includes a flange (98) that aligns to a magnetic surface of said base (82) and including a plurality of magnetic surfaces mounted to said flange (98) and aligned to magnetically couple to a magnetically permeable ring (88).

15. A valve as set forth in claim 13 or 14, **characterized by** a resilient member (116) mounted to resiliently bias said valve piece (114).

16. A valve as set forth in one of claims 13 to 15, **characterized in that** an adjustable member (120) mounts to said valve piece (114) to vary a range of movement of said adjustable member (120).

17. A valve as set forth in one of claims 13 to 16, **characterized in that** the first seal includes a first O'ring seal (92, 94), the magnetic portion (88) includes an annular magnetically permeable surface and the magnetic portion (106) of the insert (90) includes a plurality of magnetically permeable surfaces aligned to couple to the magnetically permeable surface of the base (82).

18. A valve as set forth in one of claims 13 to 17, **characterized in that** an adjuster (120) and a resilient member (116) are mounted to adjustably bias a surface of said valve piece (114) into sealing engagement with the second seal (110).

## Patentansprüche

1. Ventil, das folgendes umfasst,
a) eine Basis (20), die i) einen zylindrischen Körper, durch den sich eine Bohrung (22) erstreckt, ii) eine erste Dichtfläche, die innerhalb der Bohrung (22) angeordnet ist, und iii) eine gewindelose Schnappfläche aufweist;
b) einen Einsatz (34), der einen Körper aufweist, der eingerichtet ist, um in der Bohrung (22) der Basis (20) eingesetzt zu werden und mit der ersten Dichtfläche zusammen zu wirken, wobei der Einsatzkörper eine Durchflussbohrung (50) beinhaltet, die eine zweite Dichtfläche innerhalb der Durchflussbohrung (50) aufweist, wobei ein Verschlussmittel, das an den Einsatzkörper angepasst ist, mit der gewindelosen Schnappfläche zusammenpasst, die einen Flansch (30), der von dem Basiskörper hervorsteht, und eine Vertiefung (32) beinhaltet, die von dem Flansch (30) beabstandet ist, und wobei das Verschlussmittel einen Arm (33) beinhaltet, der eine Flanschfläche (70) aufweist und angeordnet ist, um sich so relativ zu dem Einsatzkörper zu bewegen, dass die Flanschfläche (70) mit der Vertiefung (32) an dem Körper zusammenpasst, wobei der Einsatz (34) in die Basis (20) eingeführt, in ihr gehalten und aus ihr entfernt werden kann durch geeignete Handhabung des Arms (33);
c) ein Ventilstück (36), das einen Schaft (48) aufweist, um in die Bohrung (50) des Einsatzes (34) eingesetzt zu werden, und wobei eine Oberfläche des Ventilstückes (36) in Form eines dichtenden Eingriffes mit der zweiten Dichtfläche zusammenpasst und das Ventilstück (36) aus dem Kontakt mit der zweiten Dichtfläche gedrückt werden kann, um so eine Passage durch die Durchflussbohrung (50) zu öffnen.

2. Ventil nach Anspruch 1, **gekennzeichnet durch** eine Mehrzahl von elastischen Elementen (44), die an dem Einsatz (34) für einen Haltezustand in dem Körper angeordnet sind und das Ventilstück (36) in einen dichtenden Eingriff mit der zweiten Dichtfläche vorspannen.

3. Ventil nach einem der Ansprüche 1 oder 2, **gekennzeichnet durch** eine Mehrzahl von Federn (44), die den Einsatz (34) in der Bohrung (22) des Basiskörpers und das Ventilstück (36) in der Bohrung (50) des Einsatzkörpers vorspannen.

4. Ventil nach einem der Ansprüche 1 oder 3, **gekennzeichnet durch** eine Feder (68), die angeordnet ist, um den Arm (33) elastisch vorzuspannen, so dass er verschwenkt, wenn der Einsatz (34) in der Bohrung (22) der Basis (20) eingesetzt wird, und in einen Haltezustand zurückkehrt, sobald die Flanschfläche (70) an der Vertiefung (32) ausgerichtet ist.

5. Ventil nach einem der Ansprüche 1 bis 4, **dadurch gekennzeichnet, dass** erste und zweite Arme (33) elastisch vorgespannt sind, um sich zu biegen und in einen Haltezustand zurückzukehren, wenn die Flanschflächen (70) der ersten und zweiten Arme (33) in die Vertiefung (32) eingreifen.

6. Ventil nach einem der Ansprüche 1 bis 5, **dadurch gekennzeichnet, dass** die ersten und zweiten Dichtungen (42, 58) O-Ringe aufweisen.

7. Ventil nach einem der Ansprüche 1 bis 6, **dadurch gekennzeichnet, dass** ein Befestigungsflansch (24) ringförmig ist und einen Durchmesser aufweist, der größer ist als ein Durchmesser des Basiskörpers.

8. Ventil nach Anspruch 7, **dadurch gekennzeichnet, dass** der Basiskörper eine ringförmige Nut (32) aufweist, wobei der Einsatz (34) erste und zweite elastisch vorgespannte Arme (33) aufweist, die vorgespannt sind, um flexibel zu schwenken und in eine Haltestellung zurückzukehren, wenn Flanschflächen (70) der ersten und zweiten Arme (33) in die Nut (32) eingreifen und so den Einsatz an dem Körper halten.

9. Ventil nach einem der Ansprüche 1 bis 8, **dadurch gekennzeichnet, dass** die erste Dichtfläche eine O-Ring-Dichtung (42) ist, die innerhalb der Bohrung (22) des Körpers der Basis (20) befestigt ist und dass die zweite Dichtfläche eine zweite O-Ring-Dichtung (58) beinhaltet, die innerhalb der Durchflussbohrung (50) befestigt ist.

10. Ventil nach Anspruch 9, **gekennzeichnet durch** eine Feder (44) zum Vorspannen des Ventilteiles (36) und zur Anlage an dem zweiten O-Ring (58).

11. Ventil nach einem der Ansprüche 1 bis 10, **dadurch gekennzeichnet, dass** die Vertiefung eine Nut (32) beinhaltet.

12. Ventil nach einem der Ansprüche 10 oder 11, **gekennzeichnet durch** eine Mehrzahl von elastischen Elementen (44), die den Einsatz (34) in die Haltestellung in den Körper vorspannen und das Ventilstück (36) in einen dichtenden Eingriff mit der zweiten O-Ring-Dichtung (58) vorspannen.

13. Ventil, das folgendes umfasst:
a) eine Basis (82), die (i) einen Körper, durch den sich eine Bohrung (96) erstreckt, und ii) eine erste Dichtung (92, 94) aufweist, die in der Bohrung (96) befestigt ist;
b) einen Einsatz (90), der einen Körper hat, der eingerichtet ist, in der Bohrung (96) der Basis (82) angeordnet zu werden und in die erste Dichtung (92, 94) einzugreifen, wobei der Einsatzkörper die Durchflussbohrung (112) beinhaltet, die eine zweite Dichtung (110) aufweist, die in der Durchflussbohrung (112) angeordnet ist; und
c) ein Ventilstück (114), das einen Schaft aufweist, der eingerichtet ist, um in der Bohrung (112) des Einsatzes (90) angeordnet zu werden, wobei eine Oberfläche des Ventilstückes (114) in dichtendem Eingriff mit der zweiten Dichtung (110) zusammenpasst, wobei die Basis einen magnetischen Anteil (88) hat und der Einsatz einen magnetischen Anteil (106) hat, wobei der magnetische Anteil (106) des Einsatzes (90) angeordnet ist, um mit dem magnetischen Anteil (88) der Basis zusammenzuwirken und den Einsatz (90) mit der Basis (82) zu koppeln, wodurch der Einsatz (90) in die Basis (82) eingesetzt, in ihr gehalten und aus ihr entfernt werden kann durch geeignetes Überwinden der magnetischen Anziehung zwischen der gekoppelten Basis (82) und dem Einsatz (90).

14. Ventil nach Anspruch 13, **dadurch gekennzeichnet, dass** der Einsatz (90) einen Flansch (98) beinhaltet, der mit einer magnetischen Oberfläche der Basis (82) fluchtet und eine Mehrzahl von magnetischen Oberflächen beinhaltet, die an dem Flansch (98) angeordnet und ausgerichtet sind, um magnetisch an einen magnetisch permeablen Ring (88) zu koppeln.

15. Ventil nach Anspruch 13 oder 14, **gekennzeichnet durch** ein elastisches Element (116), das angeordnet ist, um das Ventilstück (114) elastisch vorzuspannen.

16. Ventil nach einem der Ansprüche 13 bis 15, **dadurch gekennzeichnet, dass** ein einstellbares Element (120) an dem Ventilstück (114) befestigt ist, um den Bereich der Bewegung des einstellbaren Elementes (120) zu variieren.

17. Ventil nach einem der Ansprüche 13 bis 16, **dadurch gekennzeichnet, dass** die erste Dichtung eine erste O-Ring-Dichtung (92, 94) beinhaltet, wobei der magnetische Anteil (88) eine ringförmige magnetisch permeable Oberfläche beinhaltet und der magnetische Anteil (106) des Einsatzes (90) eine Mehrzahl von magnetisch permeablen Oberflächen beinhaltet, die ausgerichtet sind, um mit der magnetisch permeablen Oberfläche der Basis (82) zu koppeln.

18. Ventil nach einem der Ansprüche 13 bis 17, **dadurch gekennzeichnet, dass** ein Einsteller (120) und ein elastisches Element (116) angeordnet sind, um eine Oberfläche des Ventilstückes (114) einstellbar in dichtendem Eingriff mit der zweiten Dichtung (110) vorzuspannen.

## Revendications

1. Valve comprenant
a) une base (20) ayant i) un corps cylindrique à travers lequel s'étend un perçage (22), ii) une première surface d'étanchéité située à l'intérieur dudit perçage (22), et iii) une surface de blocage dépourvue de pas de vis ;
b) un insert (34) ayant un corps adapté à être monté dans le perçage (22) de ladite base (20) et à engager ladite première surface d'étanchéité, dans lequel ledit corps d'insert inclut un perçage d'écoulement (50) ayant une seconde surface d'étanchéité à l'intérieur du perçage d'écoulement (50), dans lequel des moyens de fixation (33) montés sur le corps d'insert sont accouplés avec la surface de blocage dépourvue de pas de vis qui inclut une bride (30) dépassant radialement du corps de la base et une dépression (32) déplacée par rapport à ladite bride (30), et dans lequel les moyens de fixation incluent un bras (33) ayant une surface en bride (70) et étant monté pour se déplacer par rapport au corps d'insert de telle façon que la surface en bride (70) s'accouple avec la dépression (32) sur ledit corps, grâce à quoi ledit insert (34) peut être inséré, retenu et extrait hors de ladite base (20) lors d'une manipulation appropriée dudit bras (33) ;
c) une pièce de valve (36) ayant une tige (48) pour être montée dans le perçage (50) dudit insert (34), et dans laquelle une surface de ladite pièce de valve (36) est accouplée en engagement étanche avec la seconde surface d'étanchéité, et ladite pièce de valve (36) peut être enfoncée hors de contact avec ladite seconde surface d'étanchéité de manière à ouvrir un passage à travers le perçage d'écoulement (50).

2. Valve selon la revendication 1, **caractérisée par** une pluralité d'éléments élastiques (44) montés sur ledit insert (34) à une condition de rétention dans ledit corps et sollicitant ladite pièce de valve (36) vers ledit engagement étanche avec ladite seconde surface d'étanchéité.

3. Valve selon l'une des revendications 1 ou 2, **caractérisée par** une pluralité de ressorts (44) montés pour solliciter ledit insert (34) dans le perçage (22) du corps de base et ladite pièce de valve (36) dans le perçage (50) du corps d'insert.

4. Valve selon l'une des revendications 1 ou 3, **caractérisée par** un ressort (68) monté pour solliciter élastiquement ledit bras (33) afin de pivoter lorsque ledit insert (34) est monté dans le perçage (22) de ladite base (20) et ramener ledit bras (33) vers une condition de rétention une fois que ladite surface en bride (70) est alignée vers ladite dépression (32).

5. Valve selon l'une des revendications 1 à 4, **caractérisée en ce que** ledit premier et ledit second bras (33) sont sollicités élastiquement pour fléchir et retourner à une condition de rétention quand les surfaces en bride (70) dudit premier et dudit second bras (33) engagent ladite dépression (32).

6. Valve selon l'une des revendications 1 à 5, **caractérisée en ce que** ledit premier et ledit second joint (42, 58) comprennent des joints toriques.

7. Valve selon l'une des revendications 1 à 6, **caractérisée en ce qu'**une bride de montage (24) est annulaire et présente un diamètre supérieur à un diamètre du corps de base.

8. Valve selon la revendication 7, **caractérisée en ce que** le corps de base inclut une gorge annulaire (32), et dans laquelle ledit insert (34) inclut un premier et un second bras (33) sollicités élastiquement, qui sont sollicités pour pivoter en flexion et retourner vers une condition de rétention quand les surfaces en bride (70) dudit premier et dudit seconde bras (33) engagent ladite gorge (32) et restreignent ainsi ledit insert (34) sur ledit corps.

9. Valve selon l'une des revendications 1 à 8, **caractérisée en ce que** la première surface d'étanchéité est un premier joint torique (42) monté à l'intérieur du perçage (22) du corps de la base (20), et **en ce que** la seconde surface d'étanchéité inclut un second joint torique (58) monté à l'intérieur du perçage d'écoulement (50).

10. Valve selon la revendication 9, **caractérisée par** un ressort (44) pour solliciter ladite pièce de valve (36) et pour venir buter contre ledit second joint torique (58).

11. Valve selon l'une des revendications 1 à 10, **caractérisée en ce que** la dépression inclut une gorge (32).

12. Valve selon la revendication 10 ou 11, **caractérisée par** une pluralité d'éléments élastiques (44) sollicitant ledit insert (34) vers une condition de rétention dans ledit corps et sollicitant ladite pièce de valve (36) en engagement étanche avec ledit second joint torique (58).

13. Valve comprenant
a) une base (82) ayant i) un corps à travers lequel s'étend un perçage (96), et ii) un premier joint (92, 94) monté dans ledit perçage (96) ;
b) un insert (90) ayant un corps adapté à être monté dans le perçage (96) de ladite base et à engager ledit premier joint (92, 94), dans lequel ledit corps d'insert inclut le perçage d'écoulement (112) ayant un second joint (110) monté à l'intérieur dans le perçage d'écoulement (112) ; et
c) une pièce de valve (114) ayant une tige adaptée à être montée dans le perçage (112) dudit insert (90) et dans laquelle une surface de ladite pièce de valve (114) est accouplée en engagement étanche avec le second joint (110), dans laquelle la base comprend une portion magnétique (88),
l'insert comprend une portion magnétique (106) telle que la portion magnétique (106) de l'insert (90) est montée pour coopérer avec la portion magnétique (88) de la base et coupler ledit insert (90) à ladite base (82), grâce à quoi ledit insert (90) peut être inséré, retenu et extrait hors de ladite base (82) en surmontant de façon appropriée l'attraction magnétique entre la base (82) et l'insert (90) accouplé.

14. Valve selon la revendication 13, **caractérisée en ce que** ledit insert (90) inclut une bride (98) qui est alignée avec une surface magnétique de ladite base (82) et incluant une pluralité de surfaces magnétiques montées sur ladite bride (98), et alignée pour être magnétiquement couplée à une bague (88) présentant une perméabilité magnétique.

15. Valve selon la revendication 13 ou 14, **caractérisée par** un élément élastique (116) monté pour solliciter élastiquement ladite pièce de valve (114).

16. Valve selon l'une des revendications 13 à 15, **caractérisée en ce qu'**un élément ajustable (120) est monté sur ladite pièce de valve (114) pour faire varier une plage de mouvement dudit élément ajustable (120).

17. Valve selon l'une des revendications 13 à 16, **caractérisée en ce que** le premier joint inclut un premier joint torique (92, 94), la portion magnétique (88) inclut une surface annulaire présentant une perméabilité magnétique, et la portion magnétique (106) de l'insert (90) inclut une pluralité de surfaces présentant une perméabilité magnétique alignées pour être accouplées à la surface de la base (82) présentant une perméabilité magnétique.

18. Valve selon l'une des revendications 13 à 17, **caractérisée en ce qu'**un dispositif d'ajustement (120) et un élément élastique (116) sont montés pour solliciter de manière ajustable une surface de ladite pièce de valve (114) en engagement étanche avec le second joint (110).
